# FASCICULE DE BREVET EUROPEEN

(11) **EP 1 337 269 B1**
(45) Date de publication et mention de la délivrance du brevet: **08.11.2006**
(21) Numéro de dépôt: 01996380.0
(22) Date de dépôt: 14.11.2001
(51) Int. Cl.: A61K 38/55, A61K 31/54, A61K 31/05, A61P 25/00, A61P 27/00

(54) **ASSOCIATION D'INHIBITEURS DE CALPAINE ET DE PIEGEURS DES FORMES REACTIVES DE L'OXYGENE**
ASSOZIATION VON CALPAINE UND INHIBITOREN VON REAKTIVE-SAUERSTOFF FREI RADIKALEN
ASSOCIATION OF CALPAIN INHIBITORS AND REACTIVE OXYGEN SPECIES TRAPPING AGENTS

(30) Priorité: 15.11.2000 FR 0014690
(43) Date de publication de la demande: 27.08.2003
(73) Titulaire: SOCIETE DE CONSEILS DE RECHERCHES ET D'APPLICATIONS SCIENTIFIQUES S.A., F-75016 Paris (FR)
(72) Inventeur: CHABRIER DE LASSAUNIERE, Pierre-Etienne, F-75016 Paris (FR); PIGNOL, Bernadette, F- 75012 Paris (FR); AUVIN, Serge, 91240 Saint-Michel-Sur-Orge (FR)
(74) Mandataire: Bourgouin, André
(86) Numéro de dépôt international: PCT/FR2001/003558
(87) Numéro de publication internationale: WO 2002/040016

(56) Documents cités:
- WO-A-01/32654
- FR-A- 2 791 571
- US-A- 5 760 048
- PATENT ABSTRACTS OF JAPAN vol. 2000, no. 10, 17 novembre 2000 (2000-11-17) & JP 2000 191616 A (SENJU PHARMACEUT CO LTD), 11 juillet 2000 (2000-07-11)
- KIM S.-J. ET AL: "Regulation of reactive oxygen species and stress fiber formation by calpeptin in Swiss 3T3 fibroblasts." CELLULAR SIGNALLING, (2002) 14/3 (205-210)., XP001064961
- BANIK, N. L. ET AL: "Role of calpain in spinal cord injury: effects of calpain and free radical inhibitors" ANN. N. Y. ACAD. SCI. (1998), 844(NEUROCHEMISTRY OF DRUGS OF ABUSE), 131-137, XP008000950
- BLUMENTHAL E J ET AL: "CHANGES IN PULMONARY CALPAIN ACTIVITY FOLLOWING TREATMENT OF MICE WITH BUTYLATED HYDROXYTOLUENE" ARCHIVES OF BIOCHEMISTRY AND BIOPHYSICS, NEW YORK, US, US, vol. 256, no. 1, juillet 1987 (1987-07), pages 19-28, XP001014555 ISSN: 0003-9861
- WANG K K W ET AL: "AN ALPHA-MERCAPTOACRYLIC ACID DERIVATIVE IS A SELECTIVE NONPEPTIDE CELL-PERMEABLE CALPAIN INHIBITOR AND IS NEUROPROTECTIVE" PROCEEDINGS OF THE NATIONAL ACADEMY OF SCIENCES OF USA, NATIONAL ACADEMY OF SCIENCE. WASHINGTON, US, vol. 93, no. 13, 25 juin 1996 (1996-06-25), pages 6687-6692, XP002053376 ISSN: 0027-8424
- PERRIN C ET AL: "MISE EN EVIDENCE DES FORMES RADICALAIRES SECONDAIRES ET ROLE DE LA CALPAINE DANS LE ALTERATIONS FOBCTIONNELLES ASSOCIEES A LA SEQUENCE ISCHEMIE-REPERFUSION MYOCARDIQUE DETECTION OF SECONDARY FREE RADICALS AND ROLE OF CALPAIN ON MYOCARDIAL POST-ISCHEMIC RECOVERY" ARCHIVES DES MALADIES DU COEUR ET DES VAISSEAUX, J.B. BALLIERE, PARIS, FR, vol. 93, no. 8, août 2000 (2000-08), pages 931-936, XP001014501 ISSN: 0003-9683
- GUTTMANN R P ET AL: "OXIDATIVE STRESS INHIBITS CALPAIN ACTIVITY IN SITU" JOURNAL OF BIOLOGICAL CHEMISTRY, AMERICAN SOCIETY OF BIOLOGICAL CHEMISTS, BALTIMORE, MD, US, vol. 273, no. 21, 22 mai 1998 (1998-05-22), pages 13331-13338, XP001015270 ISSN: 0021-9258

## Description

La présente invention concerne une composition pharmaceutique comprenant, à titre de principe actif, au moins une substance inhibitrice des calpaïnes et au moins une substance piégeur des formes réactives de l'oxygène (ROS pour *"reactive oxygen species"),* et éventuellement un support pharmaceutiquement acceptable. L'invention concerne également un produit comprenant au moins une substance inhibitrice de calpaïnes et au moins une substance piégeur de formes réactives de l'oxygène, de manière sélective ou non, en tant que produit de combinaison, sous forme séparée, de ces principes actifs.

Compte tenu du rôle potentiel des calpaïnes et des ROS en physiopathologie, une composition selon l'invention peut produire des effets bénéfiques ou favorables dans le traitement de pathologies où ces enzymes et / ou ces espèces radicalaires sont impliquées, et notamment :
- les maladies inflammatoires et immunologiques comme par exemple l'arthrite rhumatoïde, les pancréatites, la sclérose en plaques, les inflammations du système gastro-intestinal (colite ulcérative ou non, maladie de Crohn),
- les maladies cardio-vasculaires et cérébro-vasculaires comprenant par exemple l'hypertension artérielle, le choc septique, les infarctus cardiaques ou cérébraux d'origine ischémique ou hémorragique, les ischémies ainsi que les troubles liés à l'agrégation plaquettaire,
- les troubles du système nerveux central ou périphérique comme par exemple les maladies neurodégénératives où l'on peut notamment citer les traumatismes cérébraux
ou de la moëlle épinière, l'hémorragie sub arachnoïde, l'épilepsie, le vieillissement, les démences séniles, y compris la maladie d'Alzheimer, la chorée de Huntington, la maladie de Parkinson, les neuropathies périphériques,
- l'ostéoporose,
- les dystrophies musculaires, la cachexie,
- les maladies prolifératives comme par exemple l'athérosclérose ou la resténose,
- la perte d'audition,
- la cataracte,
- les transplantations d'organes,
- les maladies auto-immunes et virales comme par exemple le lupus, le sida, les infections parasitaires et virales, le diabète et ses complications, la sclérose en plaques,
- le cancer,
- toutes les pathologies caractérisées par une production excessive des ROS et / ou une activation des calpaïnes.

Dans l'ensemble de ces pathologies, il existe des évidences expérimentales démontrant l'implication des ROS (Free Radic. Biol. Med. (1996) 20, 675-705 ; Antioxid. Health. Dis. (1997) 4 (Handbook of Synthetic Antioxidants), 1-52) ainsi que l'implication des calpaïnes (Trends Pharmacol. Sci. (1994) 15, 412419 ; Drug News Perspect (1999) 12, 73-82). A titre d'exemple, les lésions cérébrales associées à l'infarctus cérébral ou au traumatisme crânien expérimental sont réduites par des agents antioxydants (Acta. Physiol. Scand. (1994) 152, 349-350; J. Cereb. Blood Flow Metabol. (1995) 15, 948-952 ; J Pharmacol Exp Ther (1997) 2, 895-904) ainsi que par des inhibiteurs de calpaïnes (Proc Natl Acad Sci U S A (1996) 93, 3428-33 ; Stroke, (1998) 29, 152-158 ; Stroke (1994) 25, 2265-2270). Le brevet US5760048 décrit l'acide 3-(4-iodophényl)-2-mercapto-2-propanoïque comme inhibiteurs de calpaïnes. La demande JP 2000191616 décrit des composés à double activité, inhibitrice de calpaïnes et inhibiteurs de radicaux libres. Mais aucune association à but thérapeutique de ces deux principes actifs, à savoir une substance inhibitrice des calpaïnes et une substance piégeur des formes réactives de l'oxygène, n'a été décrite dans la littérature. De plus ces deux principes actifs agissent de façon synergique.

La présente invention a donc pour objet une composition pharmaceutique comprenant, à titre de principe actif, au moins une substance inhibitrice des calpaïnes et au moins une substance piégeur des formes réactives de l'oxygène, et éventuellement un support pharmaceutiquement acceptable. Dans la présente demande, l'expression "au moins une substance inhibitrice de calpaïnes et au moins une substance piègeurs de radicaux libres" signifie l'association d'au moins deux entités différentes, l'une inhibiteur de calpaïnes et l'autre piégeurs de radicaux libres.

La présente invention a plus particulièrement pour objet une composition pharmaceutique comprenant, à titre de principe actif, une substance inhibitrice des calpaïnes et une substance piégeur des formes réactives de l'oxygène, et éventuellement un support pharmaceutiquement acceptable.

Dans le terme piégeur de formes réactives de l'oxygène, il faut comprendre toute substance chimique ou enzymatique capable de s'opposer ou de piéger les ou l'une des formes réactives de l'oxygène telles que O₂^{•-}, OH^{•}, RO₂^{•}, RO^{•}, ONO₂⁻, NO, ^{•}NO₂^{•} ou H₂O₂ (Halliwell B., Gutteridge JMC., Free radicals in biology and medicine, 2^{nd} ed., Oxford, Clarendon Press, 1989). Ces substances peuvent être naturelles ou synthétiques et posséder des propriétés antioxydantes. (Santrucek and Krepelka, Antioxidants - Potential chemotherapeutic agents Drugs Future 13, 975-996, 1988 ; Jackson et al, Antioxidants : a biological defense mechanism for the prevention of atherosclerosis, Med. Res. Reviews 13, 161-182 (1993) ; Aruoma, Characterization of drugs as antioxidant prophylactics, Free Rad. Biol. Med. 20, 675-705 (1996)). L'activité anti-oxydante d'une substance chimique peut être déterminée par des tests pharmaceutiques classiques tels que ceux illustrés dans la partie expérimentale de la présente demande. Une telle substance anti-oxydante peut ainsi présenter, sur la peroxydation lipidique (cf test dans la partie expérimentale de la présente demande), une CI₅₀ inférieure à 100 µM et de préférence inférieure à 10 µM.

Les calpaïnes sont des enzymes cytosoliques protéolytiques qui appartiennent à la famille des cystéines protéases. L'activation des calpaïnes est strictement dépendante du calcium (Suzuki et al. Calpain : Novel family members, activation, and physiologicalfonction (1995) Biol.Chem, Hoppe-Seyler,376, 523-529). Dans des conditions physiologiques normales, ces enzymes ne sont pas ou peu activées. Au contraire l'élévation excessive de calcium intracellulaire qui est observée dans certains phénomènes physiopathologiques induit une forte activation des calpaïnes. Cette activation entraîne le clivage sélectif et par conséquent la perte de fonction de protéines du cytosquelette, de récepteur membranaire ou de facteurs de transcription conduisant à la mort cellulaire et à la dégradation des tissus. L'activation des calpaïnes apparaît jouer un rôle majeur et délétère dans plusieurs pathologies comme par exemple les maladies neurodégénératives, l'ischémie cérébrale ou les dystrophies musculaires, que pourraient éventuellement corriger des inhibiteurs de ces enzymes (Wank K et al. Calpain inhibition : an overview of its therapeutical potential (1994) Trends in Pharmacological Sciences 15, 412-419). L'activité inhibitrice d'une substance chimique vis à vis des calpaïnes peut être déterminée par des tests pharmaceutiques classiques tels que ceux illustrés dans la partie expérimentale de la présente demande Une telle substance inhibitrice de calpaïne peut ainsi présenter, sur l'inhibition de la calpaïne (cf test dans la partie expérimentale de la présente demande), une CI₅₀ inférieure à 10 µM.

L'invention a également pour objet un produit comprenant au moins une substance inhibitrice des calpaïnes et au moins une substance piégeur de formes réactives de l'oxygène en tant que produit de combinaison, sous forme séparée, pour une utilisation simultanée ou séquentielle dans le traitement de pathologies dans lesquelles les calpaïnes et les formes réactives de l'oxygène sont impliquées, pathologies telles que les maladies inflammatoires et immunologiques, les maladies cardio-vasculaires et cérébro-vasculaires, les troubles du système nerveux central, l'ostéoporose, les dystrophies musculaires, la cachexie, la perte d'audition, les maladies prolifératives, la cataracte, les transplantations d'organes, les maladies auto-immunes et virales, le cancer et toutes les pathologies caractérisées par une production excessive des ROS et / ou une activation des calpaïnes, et de préférence, les troubles du système nerveux central ou périphérique, les dystrophies musculaires, la cachexie, la perte d'audition et la cataracte.

Dans une composition pharmaceutique ou un produit selon l'invention, l'inhibiteur des calpaïnes et le piégeur de formes réactives de l'oxygène peuvent se présenter à des doses qui peuvent être identiques ou différentes. Les dosages sont choisis en fonction des composés associés à des diluants ou excipients appropriés.

L'inhibiteur des calpaïnes et le piégeur de formes réactives de l'oxygène peuvent être administrés de manière simultanée ou séquentielle, par la même voie d'administration ou par des voies différentes. De préférence, les voies d'administration sont orale, parentérale ou topique.

Les composés phénoliques piégeurs des formes réactives de l'oxygène peuvent également être choisis parmi les composés de formule générale I_{A} 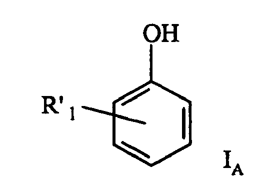 ou
dans laquelle R'₁ représente un ou plusieurs substituants choisis parmi l'atome d'hydrogène, les radicaux hydroxy, halo, carboxy, alkyle inférieure, alkoxy inférieur, alkényle inférieur ou alkoxycarbonyle, les radicaux alkyle, alkoxy et alkényles étant éventuellement substitués par un radical hydroxy, halo, carboxy ou amino ; et R'₂ représente un ou plusieurs substituants choisis parmi l'atome d'hydrogène ou les radicaux alkyle inférieur éventuellement substitué, alkoxy inférieur, hydroxy, halo, amino ou carboxy. De préférence, les composés de formule générale I_{A} sont l'acide 3-5-di-*tert*-butyl-4-hydroxybenzoïque, le 2,3,6-triméthyl-2-hexyloxy-phénol, le 2,6-di*-tert-*butyl-4-méthoxy-phénol, le 2,6-di-*tert*-butyl-4-méthyl-phénol.

Parmi les inhibiteurs des formes réactives de l'oxygène, on peut également citer les composés de formule IV_{A} ou V_{A} 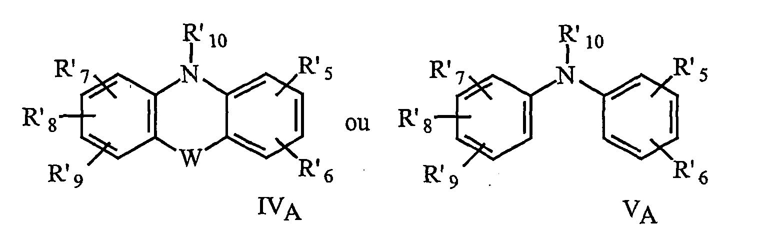 dans lequel R'₅, R'₆, R'₇, R'₈, R'₉ représentent, indépendamment, un atome d'hydrogène, un radical alkyle, alkoxy, cyano, halo, hydroxy, nitro ou -NR'₁₁R'₁₂,
R'₁₁ et R'₁₂ représentent, indépendamment, un atome d'hydrogène, un radical alkyle ou un groupe -COR'₁₃ ou bien R'₁₁ et R'₁₂ forment ensemble avec l'atome d'azote auquel ils sont rattachés un hétérocycle éventuellement substitué,
R'₁₃ représente un atome d'hydrogène, un radical alkyle, alkoxy ou -NR'₁₄R'₁₅,
R'₁₄ et R'₁₅ représentent, indépendamment, un atome d'hydrogène ou un radical alkyle, ou bien R'₁₄ et R'₁₅ forment ensemble avec l'atome d'azote auquel ils sont rattachés un hétérocycle éventuellement substitué,
R'₁₀ représente un atome d'hydrogène, un radical alkyle ou un groupe -COR'₁₆,
R'₁₆ représente un atome d'hydrogène, un radical alkyle, alkoxy ou -NR'₁₇R'₁₈,
R'₁₇ et R'₁₈ représentent, indépendamment, un atome d'hydrogène ou un radical alkyle, ou bien R'₁₇ et R'₁₈ forment ensemble avec l'atome d'azote auquel ils sont rattachés un hétérocycle éventuellement substitué,
W représente une liaison, O ou S ou encore un radical -N(R'₁₉)-, dans lequel R'₁₉ représente un atome d'hydrogène ou un radical alkyle.
composés de formule IV_{A}, on peut également citer les phénothiazines et plus particulièrement la 2-méthoxyphénothiazine, les phénoxazines et les phénazines. Comme exemples de composés de formule V_{A}, on peut citer les diphénylamines telles que la 4-hydroxydiphénylamine, la 4-aminodiphénylamine ou la 4-méthoxy-N-(4-méthoxyphényl)aniline.

Dans les définitions indiquées ci-dessus, l'expression halo représente le radical fluoro, chloro, bromo ou iodo, de préférence chloro, fluoro ou bromo. Par alkyle, lorsqu'il n'est pas donné plus de précision, ou alkyle inférieur, on entend un radical alkyle linéaire ou ramifié comptant de 1 à 6 atomes de carbone comme, par exemple, les radicaux méthyle, éthyle, propyle, isopropyle, butyle, isobutyle, sec-butyle et tert-butyle, pentyle, néopentyle, isopentyle, hexyle, isohexyle. Les radicaux alkoxy, lorsqu'il n'est pas donné plus de précision, ou alkoxy inférieur peuvent correspondre aux radicaux alkyle indiqués ci-dessus comme par exemple les radicaux méthoxy, éthoxy, propyloxy ou isopropyloxy mais également butoxy linéaire, secondaire ou tertiaire. Le terme alkylthio inférieur désigne de préférence les radicaux dans lesquels le radical alkyle est tel que défini ci-dessus comme par exemple méthylthio, éthylthio.

Par alkényle, lorsqu'il n'est pas donné plus de précision, ou alkényle inférieur, on entend un radical alkyle linéaire ou ramifié comptant de 2 à 6 atomes de carbone et présentant au moins une insaturation (une ou plusieurs double liaison). Comme exemple, on peut citer les groupes vinyle, allyle, propènyle, isopropènyle, pentènyle, butènyle, hexanyle, propènyle et butadiényle. Le terme cycloalkyle désigne de préférence les cycles cyclopropyle, cyclobutyle, cyclopentyle ou cyclohexyle.

L'expression aryle représente un radical aromatique, constitué d'un cycle ou de cycles condensés, comme par exemple le radical phényle ou naphtyle. Le terme aryloxy désigne de préférérence les radicaux dans lesquels le radical aryle est tel que défini ci-dessus comme par exemple le radical phénoxy. L'expression hétéroaryle désigne un radical aromatique, constitué d'un cycle ou de cycles condensés, avec au moins un cycle contenant un ou plusieurs hétéroatomes identiques ou différents choisis parmi le soufre, l'azote ou l'oxygène. Comme exemple de radical hétéroaryle, on peut citer les radicaux thiényle, furyle, pyrrolyle, imidazolyle, pyrazolyle, isothiazolyle, thiazolyle, isoxazolyle, oxazolyle, pyridyle, pyrazinyle, pyrimidyle, quinolinyle, benzothiényle, benzofuryle et indolyle.

Le terme hétérocycloalkyle représente de préférence un hétérocycle saturé mono ou bicyclique, comportant de 1 à 5 hétéroatomes choisis parmi O, S, N. Comme exemple, on peut citer : tétrahydrofuranne, tétrahydropyranne, oxétane, tétrahydrothiophène, tétrahydrothiopyranne, thiétane, pyrrolidine, pipéridine, azétidine, 1,3-dioxanne, 1,3-dioxolanne, 1,3-dithiolanne, 1,3-dithianne, 1,3-oxathiolanne, 1,3-oxazolidine, 1,3-imidazolidine ou 1,3-thiazolidine.

Le terme arylalkyl (ou aralkyle) désigne un radical dans lequel respectivement les radicaux aryle et alkyle sont tels que définis ci-dessus comme par exemple benzyle, phenéthyle ou naphtylméthyle. Les radicaux alkoxycarbonyle, (alkyl)aminocarbonyle et (dialkyl)aminocarbonyle désignent les radicaux dans lesquels respectivement les radicaux alkyle et alkoxy ont la signification indiquée précédemment. Les termes alkylcarbonylaminoalkyle, aryloxy alkyle, aryl alkoxy, hétéroaryle alkyle, cycloalkyl alkyle désignent les radicaux dans lesquels les radicaux alkyle, aryloxy, aryle, hétéroaryle et cycloalkyle sont tels que définis ci-dessus.

Dans le cas de radicaux de formule -NRⁱR^{j} où Rⁱ et R^{j} forment ensemble avec l'atome d'azote auquel ils sont rattachés un hétérocycle éventuellement substitué, l'hétérocycle est de préférence saturé et comprend de 4 à 7 chaînons et de 1 à 3 hétéroatomes incluant l'atome d'azote déjà présent, les hétéroatomes supplémentaires étant choisis indépendamment dans le groupe constitué des atomes O, N et S. Ledit hétérocycle peut être, par exemple, le cycle azétidine, pyrrolidine, pipéridine, pipérazine, morpholine ou thiomorpholine. Ledit hétérocycle peut être substitué par un ou plusieurs substituants identiques ou différents choisis parmi un radical alkyle, alkoxy, aryle, aralkyle, hydroxy ou halo.

Selon la définition des groupes variables, un composé de formule I_{B} tel que définie ci-dessus, peut présenter un ou plusieurs carbones asymétriques. L'invention concerne les composés de formule I_{B} telle que définie ci-dessus, composés qui peuvent se trouver sous forme racémiques, énantiomères ou diastéréoisomères.

l'invention a pour objet une composition ou un produit tel que défini ci-dessus, caractérisé en ce que l'inhibiteur de calpaïnes répond à la formule (I_{B})

T-NH-CH(R₁)-C(O)-NH-CH(R₂)-C(O)-[NH-CH(R₃)-C(O)]ₙ-Y (I_{B})

telle que définie ci-dessus et dans laquelle
R₁, R₂ et R₃ représentent, indépendamment, un radical alkyle éventuellement substitué par un ou plusieurs substituants choisis parmi : hydroxy, mercapto, akylthio inférieur, carboxy, aminocarbonyle, amino, guanidino, ou phényle, indole ou imidazole éventuellement substitué,
le ou les substituants des radicaux phényle, indole et imidazole étant choisis parmi : halo, hydroxy, alkyle inférieur ou alkoxy inférieur ;
Y représente l'atome d'hydrogène ou un radical -C(O)-Ry ou -C(O)-O-R'y dans lequel
Ry représente un radical alkyle inférieur ou -N(Ry₁)(Ry₂);
R'y représente un radical alkyle inférieur ou benzyle ;
Ry₁ représente l'atome d'hydrogène et Ry₂ représentent, indépendamment, l'atome d'hydrogène, un radical alkyle inférieur, alkoxy inférieur, arylalkyle inférieur, hétéroarylalkyle inférieur, cycloalkyl-alkyle, hétérocycloalkyle, ces radicaux pouvant être substitués par un ou plusieurs susbtituants identiques ou différents choisis parmi : halo, hydroxy, trifluorométhyle, alkyle inférieur, alkoxy inférieur, amino, (alkyl inférieur)amino, di(alkyl inférieur)amino, aryloxy, arylalkoxy ;
n représente 0 ou 1 ;
T représente un radical de formule -C(O)-O-Rt₁ ou -C(O)-Rt₂ dans laquelle
Rt₁ représente un radical alkyle inférieur, benzyle, phenéthyle ;
Rt₂ représente un radical alkyle inférieur, arylalkyle inférieur, aryloxy alkyle inférieur ou un hétéroaryle.

De manière très préférentielle, l'invention a pour objet une composition ou un produit tel que défini ci-dessus, caractérisé en ce que l'inhibiteur de calpaïnes répond à la formule (I_{B})

T-NH-CH(R₁)-C(O)-NH-CH(R₂)-C(O)-[NH-CH(R₃)-C(O)]ₙ-Y (I_{B})

telle que définie ci-dessus et dans laquelle
R₁ et R₂ représentent, indépendamment, un radical alkyle éventuellement substitué par un radical phényle, lui même éventuellement substitué par halo, hydroxy ;
Y représente l'atome d'hydrogène ;
T représente un radical de formule -C(O)-O-Rt₁ dans laquelle Rt₁ représente un radical alkyle inférieur ou benzyle, et n = 0.

De manière préférentielle, l'invention a pour objet une composition ou un produit tel que défini ci-dessus, dans lequel le piégeur de formes réactives de l'oxygène est choisi parmi l'acide 3,5-di-*tert*-butyl-4-hydroxybenzoïque, le 2,3,6-triméthyl-2-hexyloxyphénol, le 2,6-di-*tert*-butyl-4-méthoxyphénol, le 2,6-di-*tert*-butyl-4-méthylphénol, 2-méthoxy-10*H*-phénothiazine, la 4-hydroxydiphénylamine, la 4-aminodiphénylamine, la 4-méthoxy-N-(4-méthoxy phényl)aniline.

L'invention a plus particulièrement pour objet également une composition ou un produit tel que défini ci-dessus, caractérisé en ce que le piégeur des formes réactives de l'oxygène est choisi parmi les composés phénoliques, les phénothiazines et les diphénylamines. De manière préférentielle, le piégeur des formes réactives de l'oxygène est choisi parmi les composés phénoliques de formule I_{A} telle que définie ci-dessus, les phénothiazines de formule IV_{A} telle que définie ci-dessus et les diphénylamines de formule V_{A} telle que définie ci-dessus.

Plus particulièrement également, l'invention a pour objet une composition ou un produit tel que défini ci-dessus et dans lequel
le piégeur des formes réactives de l'oxygène est choisi parmi : l'acide 3,5*-*di*-tert-*butyl-4-hydroxybenzoïque (BHT), la 2-méthoxy-10*H*-phénothiazine et la 4-hydroxydiphénylamine ; et
le composé inhibiteur de calpaïnes est choisi parmi : (1S)-1-({[(1S)-1-formyl-3-méthylbutyl]amino}carbonyl)-3-méthylbutylcarbamate de benzyle (ou Z-Leu-Leu-H), (1*S*)-1-{[(1-benzyl-2-oxoéthyl)amino] carbonyl}-3-méthylbutylcarbamate de benzyle (ou Z-Leu-Phe-H), l'ester benzylique de l'acide [1-[[1-formylpentyl)amino]carbonyl]-3-methylbutyl]carbamique (calpeptine).

L'invention a enfin pour objet l'utilisation d'une substance inhibitrice des calpaïnes et d'une substance piégeur des formes réactives de l'oxygène, pour la préparation d'un médicament destiné à traiter les troubles du système nerveux central ou périphérique, les dystrophies musculaires, la cachexie, la perte d'audition et la cataracte. La substance inhibitrice des calpaïnes répond à la formule I_{B} telle que définie ci-dessus. De manière préférentielle également, le piégeur des formes réactives de l'oxygène est choisi parmi les composés phénoliques, les phénothiazines et les diphénylamines, et plus particulièrement, le piégeur des formes réactives de l'oxygène est choisi parmi les composés phénoliques de formule I_{A} telle que définie ci-dessus, les phénothiazines de formule IV_{A} telle que définie ci-dessus et les diphénylamines de formule V_{A} telle que définie ci-dessus.

Les composés inhibiteurs des calpaïnes et piégeurs de formes réactives de l'oxygène sont commerciaux ou peuvent être préparés par les méthodes connues de l'homme de l'art (ou par analogie à ces dernières) (J. Med. Chem. , vol. 37, 2918-2929 (1994) ; TIPS, vol. 11, 139-142 (1990) ; TIBS, vol. 16, 150-153 (1991) ; Br. J. Pharmacol., vol. 110, 369-377 (1993)).

Tous les termes techniques et scientifiques utilisés dans le présent texte ont la signification connue de l'homme de l'art.

### Partie expérimentale

Les exemples suivants sont présentés pour illustrer les procédures ci-dessus et ne doivent en aucun cas être considérés comme une limite à la portée de l'invention.

Soit A le piégeur de formes réactives de l'oxygène et B l'inhibiteur de calpaïnes.

### Exemple 1

Composé AB, combinaison du composé A : l'acide 3,5-di-*tert*-butyl-4-hydroxybenzoïque (BHT), antioxydant piégeur de radicaux libres oxygénés, et du composé B : Z-Leu-Leu-H, inhibiteur des calpaïnes.

### Exemple 2

Composé AB, combinaison du composé A : 2-méthoxy-10*H*-phénothiazine, antioxydant piégeur de radicaux libres oxygénés, et du composé B : Z-Leu-Phe-H inhibiteur des calpaïnes.

### Exemple 3

Composé AB, combinaison du composé A : l'acide 3,5-di-*tert*-butyl-4-hydroxybenzoïque (BHT), antioxydant piégeur de radicaux libres oxygénés, et du composé B: calpeptine, un inhibiteur de calpaïnes.

### Exemple 4

Composé AB, combinaison du composé A : la 4-hydroxydiphénylamine, antioxydant piégeur de radicaux libres oxygénés, et du composé B : Z-Leu-Leu-H, un inhibiteur de calpaïnes.

### Exemple 5

Composé AB, combinaison du composé A : la 2-méthoxy-10*H*-phénothiazine, antioxydant piégeur de radicaux libres oxygénés, et du composé B : calpeptine, un inhibiteur de calpaïnes.

### Etude pharmacologique

Les composés de l'invention ont été soumis à quelques tests biologiques *in vitro,* afin de prouver leur activité à bloquer la calpaïne et à piéger les radicaux libres. Leur activité a été évaluée sur des tests enzymatiques et sur un modèle de protection de la mort cellulaire. Dans ce modèle, la mort cellulaire par nécrose est induite par la maitotoxine (J Neurochem 1999, 72 (5) : 1853-1863, Mc Ginnis et al). La maitotoxine est une toxine marine qui active les canaux calciques (Biochem. Pharmacol 1990,39:1633-1639, Gusovsky et al). Il en résulte une augmentation de calcium intracellulaire de 10-20 fois dans les cellules gliales de rat C6 (Chem Res Toxicol 1999:12:993-1001, Konoki et al). Le traitement des neuroblastomes humains SHSY5Y par la maitotoxine induit une activation de l'activité de la calpaïne via l'augmentation de calcium intracellulaire (Archives of Biochemistry and Biophysics, 331 (2): 208-214, Wang et al). Les calpaïnes sont des cystéines protéases cytosoliques qui ont absolument besoin de calcium pour être activées. Deux isoformes de calpaïnes sont distribuées de façon ubiquitaire dans les tissus : la calpaïne I ou "µ" et la calpaïne II ou "m" qui nécessitent respectivement une concentration µM ou mM de calcium pour être activées.

Dans le modèle *in situ* de nécrose induite par la maitotoxine, une stimulation de la calpaïne et une production de formes réactives de l'oxygène se produit. L'effet partiellement protecteur sur la nécrose d'inhibiteur de calpaïnes ou de piégeur des formes réactives de l'oxygène est démontré. Les effets de l'association ont été comparés à ceux produits par un traitement avec l'inhibiteur de calpaïne ou du piégeur des formes réactives de l'oxygène seul. L'association d'un inhibiteur de calpaïnes et d'un piégeur des formes réactives de l'oxygène montre un effet protecteur significatif sur la nécrose induite par la maitotoxine par rapport à l'effet de l'inhibiteur de calpaïne ou de piégeur des formes réactives pris séparément et aux doses utilisées. Ceci prouve la synergie entre l'inhibiteur de calpaïnes et le piégeur des formes réactives de l'oxygène.

### 1) Etude des effets sur la Calpaïne I humaine

Le test consiste à mesurer l'activité de l'enzyme (enzyme purifiée à partir d'erythrocytes humains) qui est incubée dans un tampon en présence d'un substrat peptidique couplé à un fluorochrome (amino -méthylcourmarine, AMC) et du calcium. L'enzyme activée par le calcium, protéolyse le substrat et libère le fragment AMC. L'AMC libéré fluoresce à 460 nm sous une excitation à 380 nm. L' activité de l'enzyme est donc proportionnelle à la quantité de fluorescence c'est à dire de fragment AMC libre. La fluorescence (380/460 nm) est mesurée à l'aide d'un fluorimètre multipuits (Victor 2, Wallac).

Le dosage se fait en micro-plaques 96 puits à fond transparent et paroi noires dans lesquels sont distribués 10 µl par puits de substance à tester dans du DMSO 10 % , 45 µl de mélange réactionnel contenant la calpaïne I humaine à 2,2 U/ml (Calbiochem, ref : 208713), le substrat Suc Leu Tyr-AMC (Bachem, ref : I-1355) à 1,1 mM dans du tampon (Tris-HCl 110 mM; NaCl 110 mM ; EDTA 2,2 mM ; EGTA 2,2 mM ; mercaptoethanol 1,1 mM). La réaction est initiée en ajoutant 45 µl de CaCl₂ 22mM. Pour déterminer le bruit de fond, des puits témoins sans calcium sont ajoutés sur la plaque (10 µL DMSO 10 % + 45 µL de tampon avec l'enzyme et le susbtrat + 45 µL H₂O). Pour déterminer l'activité totale de l'enzyme des puits témoins sans produit sont ajoutés sur la plaque (10 µL DMSO 10 % + 45 µL de tampon avec l'enzyme et le susbtrat + 45 µL de CaCl₂ 22 mM ). Chaque concentration des produits (0,1 nM à 10 µM) est testée en duplicats. Les plaques sont agitées et l'incubation a lieu pendant une heure à 25° C à l'obscurité. La fluorescence est lue à 380/460 nm à l'aide du Victor.

Les résultats sont exprimés en valeur de CI₅₀ et sont résumés dans le tableau 1 ci-dessous.

### 2) Effet in situ sur l'activité calpaïne dans des neuroblastomes Humains (SHSY5Y) et dans des cellules gliales de rat (C6)

Les neuroblastomes humains SHSY5Y et les cellules gliales de rat C6 sont ensemencées à 50 000 et 25 000 cellules, respectivement, par puits dans des plaques à 96 puits dans du DMEM 10 % FBS. Le lendemain, les cellules qui ont adhéré sont lavées 3 fois dans du milieu DMEM sans sérum et 40 mM Hepes. Cent microlitres du produit B sont déposés dans les puits. Après une heure d'incubation à 37° C sous une atmosphère de 5 % de CO₂, 10 µl contenant le substrat fluorescent de la calpaÏne (Suc-Leu-Tyr-AMC) et la maitotoxine (Sigma, ref : M-9159), pour obtenir une concentration finale dans le puits de 100 µM et de 1 nM respectivement, sont rajoutés.

Pour déterminer l'activité totale de l'enzyme cellulaire, des puits sans produit sont ajoutés sur la plaque (100 µL DMSO 100^{ème} plus 10 µl de MTX et substrat). Les bruits de fond sont déterminés en rajoutant des puits contrôles sans MTX. Chaque concentration des produits (0,3 µM à 200 µM pour les SHSY5Y et 0,01 µM à 100 µM pour les C6) est testée en triplicats. Les plaques sont agitées, la fluorescence est lue à 380/460 nm à l'aide du Victor à T zéro. L'incubation a lieu pendant quatre heures pour les SHSY5Y et une heure trente pour les cellules C6 à 30° C à l'obscurité.

Les résultats sont exprimés en valeur de CI₅₀ et sont résumés dans le tableau 1 ci-dessous.

**Tableau 1 : activité d'inhibition enzymatique des composés A et B**

| | Inhibition calpaïne I Humaine [CI₅₀ (nM)] | | |
|---|---|---|---|
| | acellulaire | *in situ* (SHSY5Y) (n = 6) | *in situ* (C6) (n = 3) |
| LEUPEPTINE | 73 | | |
| EXEMPLE 1 | | | |
| A | - | - | - |
| B | 6 | 13370 ± 2390 | 1750 ± 340 |
| EXEMPLE 2 | | | |
| A | - | - | - |
| B | 8 | - | 2200 ± 290 |
| EXEMPLE 3 | | | |
| A | - | | |
| B | 2,8 | | |
| EXEMPLE 4 | - | | |
| A | - | | |
| B | 6 | | |
| EXEMPLE 5 | | | |
| A | - | | |
| B | 2,8 | | |

(la leupeptine ainsi que les exemples 3 à 5 n'ont été testés que sur l'inhibition de calpaïne I humaine acellulaire).

### 3) Effet in vitro sur la lipoperoxydation lipidique

Le test consiste à induire la peroxydation lipidique par les réactions de Fenton en présence de fer et d'ascorbate. Le degré de peroxydation lipidique est déterminé par la concentration en malonaldehyde (MDA). Le MDA produit par la peroxydation lipidique des acides gras insaturés est un bon indice de la peroxydation lipidique (Esterbauer et al, 1990 ; Meth. Enzymol. 186: 407-421). Pour ce test colorimétrique, la condensation d'une molécule de MDA avec deux molécules de réactif chromogène R (N-méthyl-2-phenylindole) produit un chromophore stable dont la longueur d'onde d'absorbance maximale est égale à 586 nM.

Les membranes sont préparées à partir de cortex de rat mâles Sprague Dawley de 200 à 250 g. Les animaux sont sacrifiés par décapitation et les cerveaux sont immédiatement prélevés et rincés dans du Tris HCl 20 mM pH 7,4 froid. Les cortex, disséqués et débarrassés de la substance blanche, sont alors broyés au potter de Thomas, dans du tampon Tris HCl 20 mM pH 7,4 à 4° C. Le broyat obtenu est centrifugé à faible vitesse pour éliminer les gros débris (515 g pendant 15 minutes à 4° C). Le surnageant est alors réparti dans des tubes de centrifugation en polycarbonate et centrifugé à 51 500 g pendant 25 min à 4° C. Les culots de membranes sont conservés à -80° C.

Le jour de l'expérience, les membranes congelées sont remises en suspension à la concentration de 0,5 équivalents grammes de cortex par 5 ml de tampon Tris HCl 20 mM pH 7,4 et homogénéisées à l'aide d'un combitips. Dans des plaques 96 puits " deep-well" 1 ml polypropylène à fond conique, 5 µl par puits de produit à tester ou du solvant sont mélangés avec 40 µl d'homogénat de cortex cérébral de rat par centrifugation (800 tr/min). Les plaques sont recouvertes d'une feuille d'aluminium autocollante et incubées à 37° C pendant 15 min sous agitation. La réaction de peroxidation lipidique est initiée par réaction de Fenton par l'ajout de 5 µl par puits du mélange de peroxydation contenant de l'EDTA à 1 mM, de l'acide ascorbique à 4 mM et du FeCl₂ à 1 mM, pour former du MDA. Les plaques sont centrifugées brièvement (800 tr/min) et recouvertes d'une feuille d'aluminium autocollante. Après 30 minutes d'incubation à 37° C sous agitation, 160 µl d'un mélange de révélation contenant 3 volumes du réactif chromogène R et un volume de méthanol sont rajoutés par puits. Après une agitation de 10 secondes, 40 µl d'HCl 37 % sont rajoutés par puits. La révélation du MDA formé est obtenue après une incubation des membranes pendant 3/4 h à 45° C.

La séparation des membranes et des surnageants se fait par filtration sous vide sur plaque 96 puits à fond filtrant multiscreen NA (Millipore, ref: MANANLY 50).

L'absorbance des surnageants récupérés dans les plaques 96 puits est lue à l'aide d'un photomètre.

Les résultats sont exprimés en valeur de CI₅₀ et sont résumés dans le tableau 2 ci-dessous.

### 4) Effet in situ sur le taux d'isoprostanes (8-isoPG1F2α)

La 8-isoPG1F2α fait partie d'une famille de composés dont la première étape de synthèse (péroxydation de l'acide arachidonique) se fait de manière non enzymatique en présence de radicaux libres (Morrow et coll, 1990). La mesure des taux de 8-iso PGF2α est considérée comme un marqueur fiable du stress oxydatif.

Les cellules gliales de rat C6 ont été ensemencées à 25 000 cellules par puits dans des plaques à 96 puits dans du DMEM 10 % FBS. Le lendemain, 100 µl du produit A sont déposés dans les puits. Après une heure d'incubation à 37° C sous une atmosphère de 5 % de CO₂, 10 µl contenant la maitotoxine, pour obtenir une concentration finale dans le puits de 1 nM, est rajouté. Après 3 heures d'incubation, les surnageants sont prélevés et congelés à -20° C. Les taux de 8-isoPG1F2α sont mesurés à l'aide d'un kit de dosage EIA spécifique (Cayman chemical, réf : 516351).

Les résultats sont exprimés en valeur de CI₅₀ (µM) et sont résumés dans le tableau 2 ci-dessous.

**Tableau 2 : activité d'inhibition enzymatique des composés A et B**

| | Inhibition de la Peroxidation lipidique [CI₅₀ (µM)] | Inhibition du taux d'isoprostanes *in situ* (C6) [CI₅₀ (µM)] (n = 3) |
|---|---|---|
| LEUPEPTINE | - | |
| EXEMPLE 1 | | |
| A | 3,7 | 101 |
| B | - | - |
| EXEMPLE 2 | | |
| A | 0,19 | 0,4 |
| B | - | - |
| EXEMPLE 3 | | |
| A | 3,7 | |
| B | - | |
| EXEMPLE 4 | | |
| A | 0,093 | |
| B | - | |
| EXEMPLE 5 | | |
| A | 0,19 | |
| B | - | |

(la leupeptine ainsi que les exemples 3 à 5 n'ont été testés que sur l'inhibition de la peroxydation lipidique).

### 5) Effet in vitro sur la nécrose induite par la maitotoxine

Les neuroblastomes humains SHSY5Y et les cellules gliales de rat C6 sont ensemencées à 25 000 cellules par puits dans des plaques à 96 puits dans du DMEM 10 % FBS pendant 24 h. Les myoblastes de rat L6 sont ensemencées à 1000 cellules par puits dans du DMEM 10 % FBS sur des microplaques 96 puits préalablement coatées avec de la gélatine 0,5 % PBS. Après 3 jours de culture le milieu est retiré et remplacé par un milieu de différenciation (DMEM + 10 µg/ml d'insuline +100 µg/ml de transferrine). 50 µl du produit A plus 50 µl de solvant ou 50 µl du produit B sont déposés dans les puits. Après une heure d'incubation à 37° C sous une atmosphère de 5 % de CO₂, 10 µl de maitotoxine pour obtenir une concentration finale de 0,1 à 1 nM (suivant le type cellulaire) est rajouté. Après 3 h d'incubation à 37° C sous une atmosphère de 5 % CO₂, la viabilité cellulaire est déterminée par l'ajout de 10 µl de sels de tétrazolium (wst-1, Roche, réf : 1644807). Les sels de tétrazolium sont clivés en molécules de formazan par des deshydrogénases mitochondriales. La quantité de formazan formé est directement corrélée aux nombres de cellules métaboliquement actives dans la culture. La mesure de l'absorbance du formazan est effectuée à l'aide d'un photomètre multipuits à une longueur d'onde de 420 nm contre une longueur d'onde de référence de 620 nm. Les résultats sont exprimés en pourcentage de protection de la nécrose induite par la maitotoxine 0,1 nM et sont résumés dans les tableaux 4 et 5 ci-dessous.

L'effet théorique a été obtenu en multipliant le pourcentage de cellules mortes en présence du produit A par le pourcentage de cellules mortes en présence du produit B (tableau 3). Le pourcentage de protection de la mort cellulaire induite par la MTX étant le pourcentage de cellules maintenues vivantes en présence du ou des produits.

**Tableau 3 : Détermination de l'interaction des composés A et B**

| Fraction Mortes obtenue après combinaison des composés A et B Effet Observé | Fraction Mortes obtenue par chacun des composés A et B Effet théorique | Interactions des composés A et B |
|---|---|---|
| FM (A+B) < | (FM A) x (FM B) | synergie |
| FM (A+B) = | (FM A) x (FM B) | additivité |
| FM (A+B) > | (FM A) x (FM B) | antagoniste |

Dans une première série d'expériences, la concentration du produit A dans l'exemple 1 est fixée (100 µM), le produit B est ajouté à des concentrations variant de zéro à 100 µM (Tableau 4A). Dans une deuxième série d'expériences, le produit B de l'exemple 1 est fixé à 100 µM, le produit A est ajouté à des concentrations variants de zéro à 100 µM (Tableau 4B). C'est l'association avec 100 µM de chacun des produits qui montre la plus importante synergie entre le produit A et le produit B pour protéger les cellules SHSY5Y de la nécrose induite par la MTX.

### Tableau 4 : Protection de la nécrose induite par la maitotoxine 0,1 nM sur des neuroblastomes Humains SHSY5Y (n=2)

**Tableau 4A : Interaction du composé A (BHT) (concentration fixe à 100 µM) en présence de concentrations variables du composé B (Z-Leu-Leu-H).**

| | Protection de la nécrose induite par MTX | | | | |
|---|---|---|---|---|---|
| | A | | B | | |
| | 100 µM | 12,5 µM | 25 µM | 50 µM | 100 µM |
| Composé A (100 µM) | 8,68 ± 0,3 | | | | |
| Composé B | | 6,89 ± 1,51 | 6,94 ± 0,2 | 9,01 ± 0,83 | 17,52 ± 2,69 |
| Composé A + Composé B | | 24,90 ± 0,93 | 32 ± 0,93 | 35,74 ± 5,71 | 44,07 ± 16,62 |
| Effet observé | | | | | |
| composé A + composé B | | 14,98 ± 1,1 | 15,01 ± 0,46 | 16,91 ± 1,03 | 24,68 ± 2,2 |
| Effet théorique | | | | | |

**Tableau 4B : Interaction du composé B (Z-Leu-Leu-H) fixe (100 µM) en présence de concentrations variables du composé A (BHT).**

| | Protection de la nécrose induite par MTX | | | | |
|---|---|---|---|---|---|
| | B | | A | | |
| | 100 µM | 12,5 µM | 25 µM | 50 µM | 100 µM |
| composé B (100 µM) | 17,52 ± 2,69 | | | | |
| composé A | | 5,18 ± 1,81 | 7,45 ± 1,65 | 8,87 ± 0,34 | 8,68 ± 0,3 |
| composé A + composé B | | 22,08 ± 4,49 | 27,38 ± 2,46 | 30,71 ± 11,01 | 44,07 ± 16,62 |
| Effet observé | | | | | |
| composé A + composé B | | 21,83 ± 1,06 | 23,7 ± 1,13 | 24,82 ± 2,73 | 24,68 ± 2,2 |
| Effet théorique | | | | | |

Au vu des résultats précédents, l'association de l'exemple 1 avec du produit A et du produit B à 100 µM de chacun des produits est effectuée sur les neuroblastomes humains (SHSY5Y ; Tableau 5A) et sur les cellules gliales avec 25 µM de produit A et 50 µM de produit B (C6 ; Tableau 5B) sur un plus grand nombre de cas.

### Tableaux 5 : Interaction synergique des composés A et B sur la protection de la nécrose.

**Tableau 5A :**

| Produit à 100 µM | Protection de la nécrose induite par MTX sur SHSY5Y (n = 8) |
|---|---|
| EXEMPLE 1 | |
| A | 19,48 ± 5,20 % |
| B | 12,87 ± 3,72 % |
| AB observé | 40,35 ± 6,21% */A, **/B, supérieur au théorique |
| AB théorique | 28,69 ± 6,73 % |

Les résultats du Tableau 5A, Exemple 1 montrent que le Z-Leu-Leu-H inhibiteur des calpaïnes, utilisé à la concentration de 100 µM, est inactif pour protéger efficacement les neuroblastomes Humains (SHSY5Y) de la nécrose induite par la maitotoxine. De même, l'acide 3-5-di-*tert*-butyl-4-hydroxybenzoïque utilisé comme piégeur des formes réactives de l'oxygène à la dose de 100 µM est faiblement actif (inférieur à 20 %). Par contre, l'association des deux composés protège significativement par rapport aux deux composés testés séparément. Cet effet est synergique, car le pourcentage de protection obtenue en associant les deux composés est supérieur à l'effet théorique calculé (Tableaux 3 et 5A, Exemple 1).

**Tableau 5B :**

| Produits | Protection de la nécrose induite par MTX sur C6 ^{(a)} |
|---|---|
| EXEMPLE 1 | |
| B [50µM] | 9,69 % |
| A[25µM] | 21,23% |
| A[50µM]B[25µM] observé | 43,95% |
| AB théorique | 28,59 % |
| EXEMPLE 2 | |
| A[50µM] | 6,56 ± 1,6 % |
| B[50µM] | 12,05 ± 2,82% |
| A[50µM]B[50µM] observé | 33,06 ± 7,99 % |
| AB théorique | 17,77 ± 3,68 % |
| EXEMPLE 3 | |
| B [100 µM] | 0,84% |
| A [50 µM] | 12,51 % |
| A[100µM]B[50 µM] observé | 37,77 % |
| AB théorique | 13,25 % |
| EXEMPLE 4 | |
| B [50 µM] | 4,91 % |
| A [100 µM] | 9,43 % |
| A[50µM]B[100µM] observé | 31,21 % |
| AB théorique | 13,88% |
| EXEMPLE 5 | |
| B [50 µM] | 13,03 % |
| A [100 µM] | 10,45 % |
| A[50µM]B[100 µM] observé | 62,09 % |
| AB théorique | 22,12% |

| | |
|---|---|
| (^{(a)} n = 1 pour les exemples 1 et 3 à 5 n = 3 pour l'exemple 2) | |

De même que pour le Tableau 5A, les résultats du Tableau 5B, Exemple 1 montrent que le Z-Leu-Leu-H inhibiteur des calpaïnes, utilisé à la concentration de 50 µM, est inactif pour protéger efficacement les cellules gliales de rat (C6) de la nécrose induite par la maitotoxine. De même, l'acide 3-5-di-*tert*-butyl-4-hydroxybenzoïque utilisé comme piégeur des formes réactives de l'oxygène à la dose de 25 µM est faiblement actif (de l'ordre de 20 %). Par contre, l'association des deux composés protège par rapport aux deux composés testés séparément (43 %). Cet effet est synergique, car le pourcentage de protection obtenue en associant les deux composés est supérieur à l'effet théorique calculé (Tableaux 3 et 5B, Exemple 1).

L'effet synergique d'un anti-ROS et d'un anti-calpaïne sur la protection de la nécrose induite par la MTX a été vérifié sur les cellules gliales avec un autre antioxydant et un autre inhibiteur de calpaïne (Exemple 2, Tableau 5B).

De la même façon, le 2-méthoxy-10*H*-phénothiazine, antioxydant piégeur de radicaux libres oxygénés, et le Z-Leu-Phe-H, inhibiteur des calpaïnes sont inactifs à la dose de 50 µM pour protéger les cellules gliales de rat (C6) de la nécrose induite par la maitotoxine. Par contre, l'association du 2-méthoxy-10*H*-phénothiazine avec Z-Leu-Phe-H (Tableau 5B, Exemple 2) montre une protection hautement significative par rapport aux deux composés testés séparément ainsi qu'à l'association théorique. Cet effet est significativement synergique, car le pourcentage de protection obtenue en associant les deux composés est significativement supérieur à l'effet théorique calculé (Tableaux 3 et 5B).

L'effet synergique d'autres antioxydants et d'autres inhibiteurs de calpaïne a également été testé sur les cellules gliales (Exemples 3 à 5, Tableau 5B). L'exemple 5 a été reproduit sur une autre lignée cellulaire notamment des cellules musculaires squelettiques de rat (myoblastes différenciées en myotubes ; Tableau 5C). Dans ces exemples, les produits A et B sont peu protecteurs par eux-mêmes vis à vis de la nécrose cellulaire induite par la maitotoxine, par contre, l'association des deux composés protège par rapport aux deux composés testés séparément. Ces effets sont synergiques, car le pourcentage de protection obtenue en associant les deux composés est supérieur à l'effet théorique calculé (Tableaux 3, 5B Exemples 3 à 5 et 5C Exemple 5).

**Tableau 5C :**

| Produits | Protection de la nécrose induite par MTX sur L6 Myoblastes différenciées en myotubes (cellules du muscle squelettique de rat) |
|---|---|
| EXEMPLE 5 | |
| B [100 µM] | 5,09 % |
| A [100 µM] | 10,94 % |
| A [100 µM] B [100 µM] observé | 25,05 % |
| AB théorique | 15,48 % |

## Revendications

1. Composition pharmaceutique comprenant, à titre de principe actif, au moins une substance inhibitrice des calpaïnes et au moins une substance piégeur des formes réactives de l'oxygène, et éventuellement un support pharmaceutiquement acceptable, **caractérisé en ce que**
- le piégeur des formes réactives de l'oxygène est choisi parmi :
les composés phénoliques de formule I_{A} 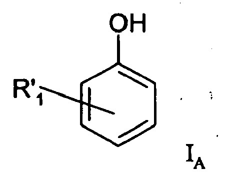 dans laquelle R'₁ représente un ou plusieurs substituants choisis parmi les radicaux carboxy et alkyle inférieure ;
les phénothiazines et les diphénylamines de formule IV_{A} ou V_{A} 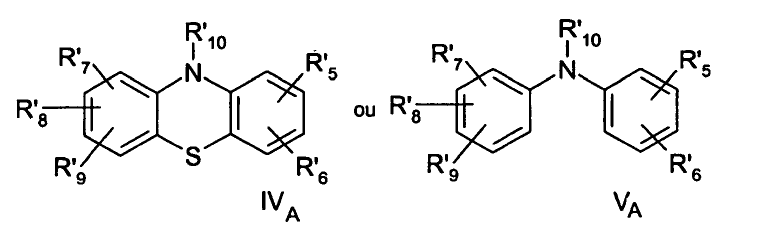 dans lequel R'₅, R'₆, R'₇, R'₈, R'₉ représentent, indépendamment, un atome d'hydrogène, un radical alkyle, alkoxy, hydroxy ;
et
- l'inhibiteur de calpaïnes répond à la formule (I_{B})
T-NH-CH(R₁)-C(O)-NH-CH(R₂)-C(O)-[NH-CH(R₃)-C(O)]ₙ-Y (I_{B})
dans laquelle
R₁ et R₂ représentent, indépendamment, un radical alkyle éventuellement substitué par un radical phényle, lui même éventuellement substitué par halo, hydroxy ;
Y représente l'atome d'hydrogène ;
T représente un radical de formule -C(O)-O-Rt₁ dans laquelle Rt₁ représente un radical alkyle inférieur ou benzyle, et n = 0,
étant entendu que les termes "alkyle", "alkyl inférieur" et "alkoxy" contiennent de 1 à 6 atomes de carbones.

2. Composition selon la revendication 1, **caractérisée en ce qu'**elle comprend, à titre de principe actif, une substance inhibitrice des calpaïnes et une substance piégeur des formes réactives de l'oxygène, et éventuellement un support pharmaceutiquement acceptable.

3. Composition selon l'une des revendications 1 à 2, **caractérisée en ce que**
le piégeur des formes réactives de l'oxygène est choisi parmi : l'acide 3,5-di*-tert-*butyl-4-hydroxybenzoïque (BHT), la 2-méthoxy-10*H*-phénothiazine et la 4-hydroxydiphénylamine ; et
le composé inhibiteur de calpaïnes est choisi parmi : (1S)-1-({[(1S)-1-formyl-3-méthylbutyl]amino}carbonyl)-3-méthylbutylcarbamate de benzyle, (1*S*)-1-{[(1-benzyl-2-oxoéthyl)amino] carbonyl}-3-méthylbutylcarbamate de benzyle et l'ester benzylique de l'acide [1-[[1-formylpentyl)amino]carbonyl]-3-methylbutyl] carbamique (calpeptine).

4. Produit comprenant au moins une substance inhibitrice des calpaïnes et au moins une substance piégeur de formes réactives de l'oxygène en tant que produit de combinaison, sous forme séparée, pour une utilisation simultanée ou séquentielle dans le traitement de pathologies choisies parmi les maladies inflammatoires et immunologiques, les maladies cardio-vasculaires et cérébro-vasculaires, les troubles du système nerveux central, l'ostéoporose, les dystrophies musculaires, la cachexie, la perte d'audition, les maladies prolifératives, la cataracte, les transplantations d'organes, les maladies auto-immunes et virales et le cancer,
**caractérisé en ce que** le piégeur des formes réactives de l'oxygène est choisi parmi les composés phénoliques de formule I_{A} 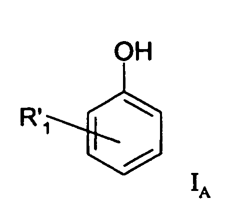 dans laquelle R'₁ représente un ou plusieurs substituants choisis parmi les radicaux carboxy et alkyle inférieure ;
les phénothiazines et les diphénylamines de formule IV_{A} ou V_{A} 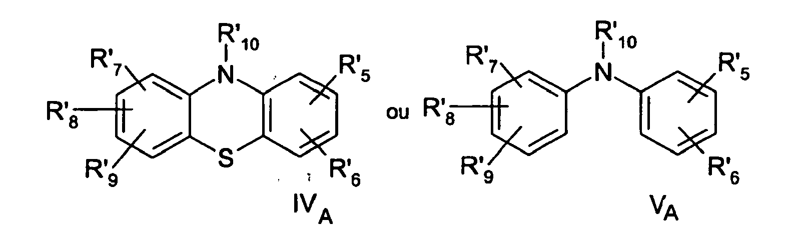 dans lequel R'₅, R'₆, R'₇, R'₈, R'₉ représentent, indépendamment, un atome d'hydrogène, un radical alkyle, alkoxy et hydroxy, et
- l'inhibiteur de calpaïnes répond à la formule (I_{B})
T-NH-CH(R₁)-C(O)-NH-CH(R₂)-C(O)-[NH-CH(R₃)-C(O)]ₙ-Y (I_{B})
dans laquelle
R₁ et R₂ représentent, indépendamment, un radical alkyle éventuellement substitué par un radical phényle, lui même éventuellement substitué par halo, hydroxy ;
Y représente l'atome d'hydrogène ;
T représente un radical de formule -C(O)-O-Rt₁ dans laquelle Rt₁ représente un radical alkyle inférieur ou benzyle, et n = 0,
étant entendu que les termes "alkyle", "alkyl inférieur" et "alkoxy" contiennent de 1 à 6 atomes de carbones.

5. Produit selon la revendication 4 dans le traitement les troubles du système nerveux central ou périphérique, les dystrophies musculaires, la cachexie, la perte d'audition, la cataracte.

6. Produit selon l'une des revendications 4 à 5, **caractérisé en ce que**
le piégeur des formes réactives de l'oxygène est choisi parmi : l'acide 3,5-di-*tert-*butyl-4-hydroxybenzoïque (BHT), la 2-méthoxy-10*H*-phénothiazine et la 4-hydroxydiphénylamine ; et
le composé inhibiteur de calpaïnes est choisi parmi : (1S)-1-({[(1S)-1-formyl-3-méthylbutyl]amino}carbonyl)-3-méthylbutylcarbamate de benzyle, (1*S*)-1-{[(1-benzyl-2-oxoéthyl)amino] carbonyl}-3-méthylbutylcarbamate de benzyle, l'ester benzylique de l'acide [1-[[1-formylpentyl)amino]carbonyl]-3-methylbutyl] carbamique (calpeptine).

7. Utilisation d'une substance inhibitrice des calpaïnes et d'une substance piégeur des formes réactives de l'oxygène choisi parmi les composés phénoliques, les phénothiazines et les diphénylamines, pour la préparation d'un médicament destiné à traiter les dystrophies musculaires, **caractérisée en ce que** la substance inhibitrice des calpaïnes répond à la formule I_{B} telle que définie à la revendication 1 et la substance piégeur des formes réactives de l'oxygène aux formules I_{A}, IV_{A} ou V_{A} telles définies à la revendication 1.

8. Utilisation d'une substance inhibitrice des calpaïnes et une substance piégeur des formes réactives de l'oxygène choisi parmi les composés phénoliques, les phénothiazines et les diphénylamines, pour la préparation d'un médicament destiné à traiter la cachexie, **caractérisée en ce que** la substance inhibitrice des calpaïnes répond à la formule I_{B} telle que définie à la revendication 1 et la substance piégeur des formes réactives de l'oxygène aux formules I_{A}, IV_{A} ou V_{A} telles définies à la revendication 1.

9. Utilisation d'une substance inhibitrice des calpaïnes et une substance piégeur des formes réactives de l'oxygène choisi parmi les composés phénoliques, les phénothiazines et les diphénylamines, pour la préparation d'un médicament destiné à traiter la perte d'audition, **caractérisée en ce que** la substance inhibitrice des calpaïnes répond à la formule I_{B} telle que définie à la revendication 1 et la substance piégeur des formes réactives de l'oxygène aux formules I_{A}, IV_{A} ou V_{A} telles définies à la revendication 1.

10. Utilisation d'une substance inhibitrice des calpaïnes et une substance piégeur des formes réactives de l'oxygène choisi parmi les composés phénoliques, les phénothiazines et les diphénylamines, pour la préparation d'un médicament destiné à traiter la cataracte, **caractérisée en ce que** la substance inhibitrice des calpaïnes répond à la formule I_{B} telle que définie à la revendication 1 et la substance piégeur des formes réactives de l'oxygène aux formules I_{A}, IV_{A} ou V_{A} telles définies à la revendication 1.

11. Utilisation d'une substance inhibitrice des calpaïnes et une substance piégeur des formes réactives de l'oxygène choisi parmi les composés phénoliques, les phénothiazines et les diphénylamines, pour la préparation d'un médicament destiné à traiter les troubles du système nerveux central ou périphérique, **caractérisée en ce que** la substance inhibitrice des calpaïnes répond à la formule I_{B} telle que définie à la revendication 1 et la substance piégeur des formes réactives de l'oxygène aux formules I_{A}, IV_{A} ou V_{A} telles définies à la revendication 1.

## Claims

1. Pharmaceutical composition comprising, as active ingredient, at least one calpain inhibitor substance and at least one reactive oxygen species trapping substance, and optionally a pharmaceutically acceptable support, **characterized in that**
- the reactive oxygen species trapping substance is selected from :
the phenolic compounds of general formula I_{A} 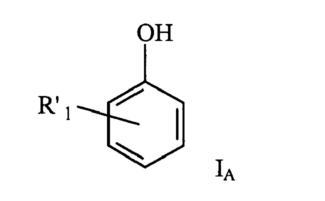 in which R'₁ represents one or more substituents chosen from carboxy and lower alkyl radicals ;
the phenothiazines and diphenylamines of formula IV_{A} or V_{A} 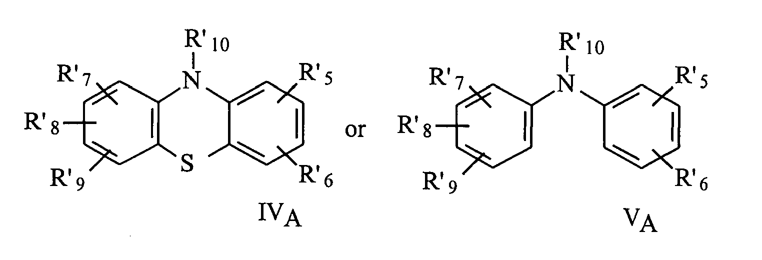 in which R'₅, R'₆, R'₇, R'₈, R'₉ represent, independently, a hydrogen atom, an alkyl, alkoxy and hydroxy radical ;
and
- the calpain inhibitor corresponds to formula (I_{B})
T-NH-CH(R₁)-C(O)-NH-CH(R₂)-C(O)-[NH-CH(R₃)-C(O)]ₙ-Y (I_{B})
in which
R₁ and R₂ represent, independently, an alkyl radical optionally substituted by a phenyl radical, itself optionally substituted by halo, hydroxy ;
Y represents the hydrogen atom ;
T represents a radical of formula -C(O)-O-Rt₁ in which Rt₁ represents a lower alkyl or benzyl radical, and n = 0,
it being understood that the terms "alkyl", lower alkyl" and "alkoxy" comprise 1 to 6 carbon atoms.

2. Composition according to claim 1, **characterized in that** it comprises, as active ingredient, a calpain inhibitor substance and a reactive oxygen species trapping substance, and optionally a pharmaceutically acceptable support.

3. Composition according to one of claims 1 to 2, **characterized in that** it comprises
a reactive oxygen species trap selected from : 3,5-di-*tert*-butyl-4-hydroxybenzoic acid (BHT), 2-methoxy-10*H*-phenothiazine and 4-hydroxydiphenylamine; and
a calpain inhibitory substance selected from benzyl (1S)-1-({[(1S)-1-formyl-3-methylbutyl]amino}carbonyl)-3-methylbutylcarbamate ; benzyl (1*S*)-1- ([(1-benzyl-2-oxoethyl)amino]carbonyl)-3-methylbutylcarbamate ; benzyl ester of [1-[[1-formylpentyl)amino]carbonyl]-3-methylbutyl]carbamic acid (calpeptine).

4. Product comprising at least one calpain inhibitory substance and at least one reactive oxygen species trapping substance as a combination product, in separated form, for simultaneous or sequential use in the treatment of pathologies selected from inflammatory and immunological diseases, cardiovascular and cerebrovascular diseases, disorders of the central nervous system, osteoporosis, muscular dystrophies, cachexia, hearing loss, proliferative diseases, cataracts, organ transplants, auto-immune and viral diseases, and cancer,
**characterized in that** the reactive oxygen species trapping substance is selected from :
the phenolic compounds of general formula I_{A} 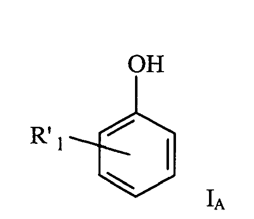 in which R'₁ represents one or more substituents chosen from carboxy and lower alkyl radicals ;
the phenothiazines and diphenylamines of formula IV_{A} or V_{A} 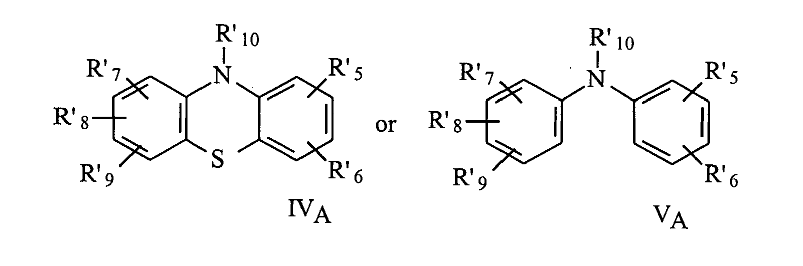 in which R'₅, R'₆, R'₇, R'₈, R'₉ represent, independently, a hydrogen atom, an alkyl, alkoxy and hydroxy radical ;
and
- the calpain inhibitor corresponds to formula (I_{B})
T-NH-CH(R₁)-C(O)-NH-CH(R₂)-C(O)-[NH-CH(R₃)-C(O)]ₙ-Y (I_{B})
in which
R₁ and R₂ represent, independently, an alkyl radical optionally substituted by a phenyl radical, itself optionally substituted by halo, hydroxy ;
Y represents the hydrogen atom ;
T represents a radical of formula -C(O)-O-Rt₁ in which Rt₁ represents a lower alkyl or benzyl radical, and n = 0,
it being understood that the terms "alkyl", lower alkyl" and "alkoxy" comprise 1 to 6 carbon atoms.

5. Product according to claim 4 in the treatment of disorders of the central or peripheral nervous system, muscular dystrophies, cachexia, hearing loss, cataracts.

6. Product according to one of claims 4 to 5, **characterized in that** it comprises
a reactive oxygen species trap selected from : 3,5-di-*tert*-butyl-4-hydroxybenzoic acid (BHT), 2-methoxy-10*H*-phenothiazine and 4-hydroxydiphenylamine; and
a calpain inhibitory substance selected from benzyl (1S)-1-({[(1S)-1-formyl-3-methylbutyl]amino}carbonyl)-3-methylbutylcarbamate ; benzyl (1*S*)-1-{[(1-benzyl-2-oxoethyl)amino]carbonyl}-3-methylbutylcarbamate ; benzyl ester of [1-[[1-formylpentyl)amino]carbonyl]-3-methylbutyl]carbamic acid (calpeptine).

7. Use of a calpain inhibitor substance and a reactive oxygen species trapping substance selected from the phenolic compounds, phenothiazines and diphenylamines, for the preparation of a medicament intended to treat muscular dystrophies, **characterized in that** the calpain inhibitor corresponds to the formula (I_{B}) as defined in claim 1 and the reactive oxygen species trapping substance to the formula I_{A}, IV_{A} or V_{A} as defined in claim 1.

8. Use of a calpain inhibitor substance and a reactive oxygen species trapping substance selected from the phenolic compounds, phenothiazines and diphenylamines, for the preparation of a medicament intended to treat cachexia, **characterized in that** the calpain inhibitor corresponds to the formula (I_{B}) as defined in claim 1 and the reactive oxygen species trapping substance to the formula I_{A}, IV_{A} or V_{A} as defined in claim 1.

9. Use of a calpain inhibitor substance and a reactive oxygen species trapping substance selected from the phenolic compounds, phenothiazines and diphenylamines, for the preparation of a medicament intended to treat hearing loss, **characterized in that** the calpain inhibitor corresponds to the formula (I_{B}) as defined in claim 1 and the reactive oxygen species trapping substance to the formula I_{A}, IV_{A} or V_{A} as defined in claim 1.

10. Use of a calpain inhibitor substance and a reactive oxygen species trapping substance selected from the phenolic compounds, phenothiazines and diphenylamines, for the preparation of a medicament intended to treat cataracts, **characterized in that** the calpain inhibitor corresponds to the formula (I_{B}) as defined in claim 1 and the reactive oxygen species trapping substance to the formula I_{A}, IV_{A} or V_{A} as defined in claim 1.

11. Use of a calpain inhibitor substance and a reactive oxygen species trapping substance selected from the phenolic compounds, phenothiazines and diphenylamines, for the preparation of a medicament intended to treat disorders of the central or peripheral nervous system, **characterized in that** the calpain inhibitor corresponds to the formula (I_{B}) as defined in claim 1 and the reactive oxygen species trapping substance to the formula I_{A}, IV_{A} or V_{A} as defined in claim 1.

## Patentansprüche

1. Pharmazeutische Zusammensetzung, umfassend als Wirkstoff mindestens eine die Calpaine hemmende Substanz und mindestens eine die reaktiven Formen des Sauerstoffs einfangende Substanz und ggf. einen pharmazeutisch annehmbaren Träger, **dadurch gekennzeichnet, dass**
- die die reaktiven Formen des Sauerstoffs einfangende Substanz ausgewählt ist aus:
den Phenolverbindungen der Formel I_{A} 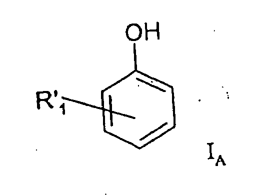 in der R'₁ einen oder mehrere Substituenten darstellt, die aus den Resten Carboxy und Niederalkyl ausgewählt sind;
den Phenothiazinen und den Diphenylaminen der Formel IV_{A} oder V_{A} 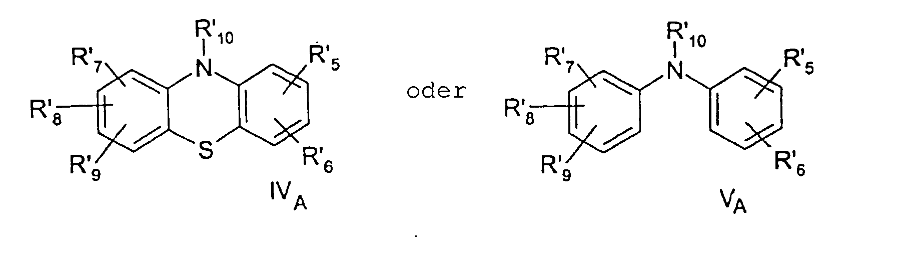
in der R'₅, R'₆, R'₇, R'₈, R'₉ unabhängig voneinander ein Wasserstoffatom, einen der Reste Alkyl, Alkoxy, Hydroxy darstellen;
und
- der Calpainhemmer der Formel (I_{B}) entspricht
T-NH-CH (R₁)-C (O)-NH-CH (R₂)-C(O)- [NH-CH(R₃) -C (O)]ₙ-Y (I_{B})
in der
R₁ und R₂ unabhängig voneinander einen Alkylrest darstellen, der ggf. mit einem Phenylrest substituiert ist, der seinerseits ggf. mit Halogen, Hydroxy substituiert ist;
Y das Wasserstoffatom darstellt;
T einen Rest der Formel -C(O)-O-Rt₁ darstellt, in der Rt₁ einen Niederalkyl- oder Benzylrest darstellt und n = 0,
wobei gilt, dass die Begriffe "Alkyl", "Niederalkyl" und "Alkoxy" 1 bis 6 Kohlenstoffatome enthalten.

2. Zusammensetzung nach Anspruch 1, **dadurch gekennzeichnet, dass** sie als Wirkstoff eine die Calpaine hemmende Substanz und eine die reaktiven Formen des Sauerstoffs einfangende Substanz und ggf. einen pharmazeutisch annehmbaren Träger umfasst.

3. Zusammensetzung nach einem der Ansprüche 1 bis 2, **dadurch gekennzeichnet, dass**
die die reaktiven Formen des Sauerstoffs einfangende Substanz ausgewählt ist aus: 3,5-Di-tert-butyl-4-hydroxybenzoesäure (BHT), 2-Methoxy-10*H*-phenothiazin und 4-Hydroxydiphenylamin; und
die Calpaine hemmende Substanz ausgewählt ist aus: (1S)-1-({[(1S)-1-Formyl-3-methylbutyl]amino}carbonyl)-3-methylbutylbenzylcarbamat, (1S)-1-{[(1-Benzyl-2-oxoethyl)amino]carbonyl}-3-methylbutylbenzylcarbamat und der Benzylester der [1-[[(1-Formylpentyl)amino]carbonyl]-3-methylbutyl]carbaminsäure (Calpeptin).

4. Produkt, umfassend mindestens eine die Calpaine hemmende Substanz und mindestens eine die reaktiven Formen des Sauerstoffs einfangende Substanz als Kombinationsprodukt in getrennter Form für eine gleichzeitige oder sequenzielle Verwendung bei der Behandlung von Krankheiten, die aus den entzündlichen und immunologischen Krankheiten, den kardiovaskulären und zerebrovaskulären Krankheiten, den Störungen des Zentralnervensystems, Osteoporose, Muskeldystrophien, Kachexie, Hörverlust, den proliverativen Krankheiten, Katarakt, Organtransplantationen, Autoimmun- und Viruserkrankungen und Krebs ausgewählt sind,
**dadurch gekennzeichnet, dass** die die reaktiven Formen des Sauerstoffs einfangende Substanz ausgewählt ist aus:
den Phenolverbindungen der Formel I_{A} 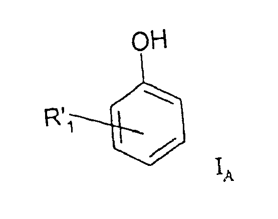
in der R'₁ einen oder mehrere Substituenten darstellt, die aus den Resten Carboxy und Niederalkyl ausgewählt sind;
den Phenothiazinen und den Diphenylaminen der Formel IV_{A} oder V_{A} 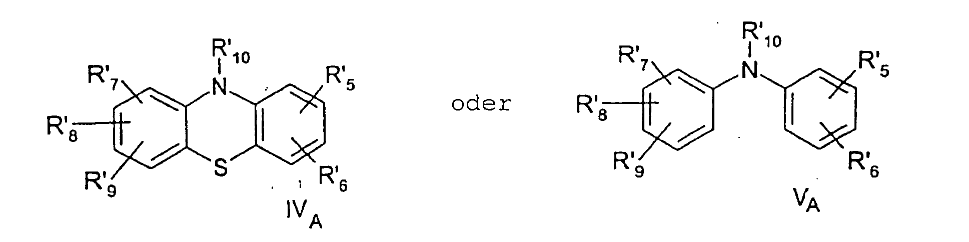
in der R'₅, R'₆, R'₇, R'₈, R'₉ unabhängig voneinander ein Wasserstoffatom, einen der Reste Alkyl, Alkoxy, Hydroxy darstellen;
und
- der Calpainhemmer der Formel (I_{B}) entspricht
T-NH-CH (R₁) -C(O)-NH-CH (R₂) -C(O)-[NH-CH (R₃)-C (O)]ₙ-Y (I_{B})
in der
R₁ und R₂ unabhängig voneinander einen Alkylrest darstellen, der ggf. mit einem Phenylrest substituiert ist, der seinerseits ggf. mit Halogen, Hydroxy substituiert ist;
Y das Wasserstoffatom darstellt;
T einen Rest der Formel -C(O)-O-Rt₁ darstellt, in der Rt₁ einen Niederalkyl- oder Benzylrest darstellt und n = 0,
wobei gilt, dass die Begriffe "Alkyl", "Niederalkyl" und "Alkoxy" 1 bis 6 Kohlenstoffatome enthalten.

5. Produkt nach Anspruch 4 für die Behandlung der Störungen des zentralen oder peripheren Nervensystems, der Muskeldystrophien, der Kachexie, des Hörverlusts, des Katarakts.

6. Produkt nach einem der Ansprüche 4 bis 5, **dadurch gekennzeichnet, dass**
die die reaktiven Formen des Sauerstoffs einfangende Substanz ausgewählt ist aus: 3,5-Di-*tert*-butyl-4-hydroxybenzoesäure (BHT), 2-Methoxy-10*H*-phenothiazin und 4-Hydroxydiphenylamin; und
die Calpaine hemmende Substanz ausgewählt ist aus: (1S)-1-({[(1S)-1-Formyl-3-methylbutyl]amino}carbonyl)-3-methylbutylbenzylcarbamat, (1S)-1-{[(1-Benzyl-2-oxoethyl)amino]carbonyl}-3-methylbutylbenzylcarbamat und der Benzylester der [1-[[(1-Formylpentyl)amino]carbonyl]-3-methylbutyl]carbaminsäure (Calpeptin).

7. Verwendung einer die Calpaine hemmenden Substanz und einer die reaktiven Formen des Sauerstoffs einfangenden Substanz, die aus den Phenolverbindungen, den Phenothiazinen und den Diphenylaminen ausgewählt ist, für die Herstellung eines Medikaments, das für die Behandlung der Muskeldystrophien bestimmt ist, **dadurch gekennzeichnet, dass** die die Calpaine hemmende Substanz der Formel I_{B} entspricht, wie sie in Anspruch 1 definiert ist, und die die reaktiven Formen des Sauerstoffs einfangende Substanz den Formeln I_{A}, IV_{A} oder V_{A} entspricht, wie sie in Anspruch 1 definiert sind.

8. Verwendung einer die Calpaine hemmenden Substanz und einer die reaktiven Formen des Sauerstoffs einfangenden Substanz, die aus den Phenolverbindungen, den Phenothiazinen und den Diphenylaminen ausgewählt ist, für die Herstellung eines Medikaments, das für die Behandlung der Kachexie bestimmt ist, **dadurch gekennzeichnet, dass** die die Calpaine hemmende Substanz der Formel I_{B} entspricht, wie sie in Anspruch 1 definiert ist, und die die reaktiven Formen des Sauerstoffs einfangende Substanz den Formeln I_{A}, IV_{A} oder V_{A} entspricht, wie sie in Anspruch 1 definiert sind.

9. Verwendung einer die Calpaine hemmenden Substanz und einer die reaktiven Formen des Sauerstoffs einfangenden Substanz, die aus den Phenolverbindungen, den Phenothiazinen und den Diphenylaminen ausgewählt ist, für die Herstellung eines Medikaments, das für die Behandlung des Hörverlusts bestimmt ist, **dadurch gekennzeichnet, dass** die die Calpaine hemmende Substanz der Formel I_{B} entspricht, wie sie in Anspruch 1 definiert ist, und die die reaktiven Formen des Sauerstoffs einfangende Substanz den Formeln I_{A}, IV_{A} oder V_{A} entspricht, wie sie in Anspruch 1 definiert sind.

10. Verwendung einer die Calpaine hemmenden Substanz und einer die reaktiven Formen des Sauerstoffs einfangenden Substanz, die aus den Phenolverbindungen, den Phenothiazinen und den Diphenylaminen ausgewählt ist, für die Herstellung eines Medikaments, das für die Behandlung des Katarakts bestimmt ist, **dadurch gekennzeichnet, dass** die die Calpaine hemmende Substanz der Formel I_{B} entspricht, wie sie in Anspruch 1 definiert ist, und die die reaktiven Formen des Sauerstoffs einfangende Substanz den Formeln I_{A}, IV_{A} oder V_{A} entspricht, wie sie in Anspruch 1 definiert sind.

11. Verwendung einer die Calpaine hemmenden Substanz und einer die reaktiven Formen des Sauerstoffs einfangenden Substanz, die aus den Phenolverbindungen, den Phenothiazinen und den Diphenylaminen ausgewählt ist, für die Herstellung eines Medikaments, das für die Behandlung der Störungen des zentralen oder peripheren Nervensystems bestimmt ist, **dadurch gekennzeichnet, dass** die die Calpaine hemmende Substanz der Formel I_{B} entspricht, wie sie in Anspruch 1 definiert ist, und die die reaktiven Formen des Sauerstoffs einfangende Substanz den Formeln I_{A}, IV_{A} oder V_{A} entspricht, wie sie in Anspruch 1 definiert sind.
